# EUROPEAN PATENT APPLICATION

(11) **EP 2 151 249 A1**
(43) Date of publication of application: **10.02.2010**
(21) Application number: 09009529.0
(22) Date of filing: 22.07.2009
(51) Int. Cl.: A61K 49/00

(54) **PH-sensitive probe comprising polymer and fluorescent dye**

(30) Priority: 28.07.2008 JP 2008193607
(71) Applicant: Canon Kabushiki Kaisha, Tokyo (JP)
(72) Inventor: Yamauchi, Fumio, Ohta-ku Tokyo (JP)
(74) Representative: Weser, Thilo

(57) **Abstract**

A polymer has a fluorescent dye sensitive to a hydrophobic environment. When a pH around the polymer decreases within a range of pH of equal to or greater than 5.5 and less than 7.4, and a secondary structure of the polymer changes from a random coil to a helix.

## Description

### BACKGROUND OF THE INVENTION

### Field of the Invention

The present invention relates to a novel polymer and a fluorescence probe having the novel polymer.

### Description of the Related Art

Detecting lesion sites such as tumors and inflammations is a very important task in biology and medicine, in particular in the field of clinical studies and internal image diagnostics. A molecular probe that can singularly detect a lesion site with high sensitivity may be necessary to perform such detection with high sensitivity and accuracy.

Lesion sites such as tumors and inflammations differ from the environment of normal tissue by a pH value. Thus, tumor or inflammation sites (pH: about 5 to about 6) are known to have a pH value lower than that of the normal tissue (pH: about 7.4). Some highly malignant tumors are even known to decrease a pH value to about 5.5. Accordingly, pH-responsive compounds are expected to be candidates for molecular probes that can detect tumors and inflammations.

Japanese Patent Publication No. 2-25138 discloses as a pH-responsive compound a fluorescent polymer indicator in which a fluorescent dye having pH reactivity is bonded to a polymer having no pH reactivity. Furthermore, Japanese Patent Publication No. 2-25138 also indicates that fluorescence intensity in a low-pH region (approximately pH 5) decreases when the environment around the indicator changes from a high-pH environment to low-pH environment.

Japanese Patent Laid-Open No. 2001-278914 discloses a polycarboxylic acid that is fluorescence labeled with anilinonaphthalene, as a fluorescence-labeled polycarboxylic acid for quantitative analysis of polycarboxylic acids used as thickening agents and stabilizers. Where such a fluorescence-labeled polycarboxylic acid is used, the surrounding environment changes from a high pH to a low pH and polycarboxylic acids form associations. As a result, a highly hydrophobic region is formed inside the association, anilinonaphthalene is taken into this region, and fluorescence is induced.

Furthermore, Japanese Patent No. 2884063 discloses a polymer having a first side chain having a polyglutamic acid and a second side chain having anilinonaphthalene, this polymer being an amphipathic polymer suitable as a means for extracting membrane proteins.

However, the fluorescence intensity of the indicator described in Japanese Patent Publication No. 2-25138 decreases with the decrease in pH. Therefore, where this indicator is to be used for detecting a low-pH site, it can be difficult to discriminate between the decrease if fluorescence intensity caused by a low pH and the decrease in fluorescence intensity caused by loss of indicator or interaction between the indicator and impurities. Thus, a low-pH site may be difficult to detect.

With the fluorescence-labeled polycarboxylic acid described in Japanese Patent Laid-Open No. 2001-278914, a highly hydrophobic region is formed mainly by association. Thus, although the acid may be reactive with a low-pH region, sufficient fluorescence intensity may not be obtained. The fluorescence-labeled polycarboxylic acid described in Japanese Patent Laid-Open No. 2001-278914 has a vinyl polymer as a backbone and, therefore, metabolism in a living body and secretion can place limitations on possible use in a living body.

In the amphipathic polymer described in Japanese Patent 2884063, the maximum fluorescence wavelength changes at pH 8 to 9. Therefore, a highly malignant tumor (pH: about 5.5) and a normal site (pH: about 7.4) can be difficult to distinguish from each other.

### SUMMARY OF THE INVENTION

One aspect of the invention relates to a polymer having a fluorescent dye sensitive to a hydrophobic environment. When a pH around the polymer decreases within a range of pH of equal to or greater than 5.5 and less than 7.4, a secondary structure of the polymer changes from a random coil to a helix, and hydrophobicity around the fluorescent dye sensitive to the hydrophobic environment increases.

Another aspect of the invention relates to a polymer having a fluorescent dye sensitive to a hydrophobic environment and a polyamino acid. When a pH around the polyamino acid decreases within a range of pH of equal to or greater than 5.5 and less than 7.4, a secondary structure of the polymer changes from a random coil to a helix, and hydrophobicity around the fluorescent dye sensitive to the hydrophobic environment increases.

Another aspect of the invention relates to a polymer comprising a fluorescent dye sensitive to a hydrophobic environment, wherein at a pH of 7.4, a probability that a secondary structure of the polymer is a random coil structure is higher than a probability that the secondary structure of the polymer is a helix structure, and at a pH of 5.5, a probability that the secondary structure of the polymer is the helix structure is higher than a probability that the secondary structure of the polymer is the random coil structure. Another aspect of the invention relates to a fluorescence probe comprising a polymer and a fluorescent dye sensitive to a hydrophobic environment,
wherein fluorescence intensity of the probe at a pH of 5.5 is higher than fluorescence intensity of the probe at a pH of 7.4 resulting from a change of a secondary structure of the polymer from a random coil structure to a helix structure.

Further features of the present invention will become apparent from the following description of exemplary embodiments with reference to the attached drawings.

### BRIEF DESCRIPTION OF THE DRAWINGS

FIG. 1 is a graph showing the pH dependence of fluorescence intensity of PGA-AN1 that is an example of a polymer in accordance with aspects of the invention and anilinonaphthalenesulfonic acid in PBS containing 10% serum.

FIG. 2 is a graph showing the pH dependence of fluorescence intensity of PGA-AN1 that is an example of the polymer in accordance with aspects of the invention and anilinonaphthalenesulfonic acid in water.

FIG. 3 is a graph showing the pH dependence of fluorescence intensity of PGA-NR that is an example of the polymer in accordance with aspects of the invention in water.

FIG. 4 is a graph showing the pH dependence of fluorescence intensity of PGA-AN-Gel that is an example of the polymer in accordance with aspects of the invention in water.

FIG. 5 is a graph showing the dependence of fluorescence intensity of PGA-AN that is an example of the polymer in accordance with aspects of the invention, anilinonaphthalenesulfonic acid, and LysoSensor^{™} Blue DND-167 in PBS.

FIG. 6 is a graph showing the pH dependence of fluorescence intensity of PGA-AN2 and PGA-AN3 that are examples of the polymer in accordance with aspects of the invention in water.

FIG. 7 is a graph showing the pH dependence of fluorescence intensity of PGA-AN4 that is an example of the polymer in accordance with aspects of the invention in water.

FIG. 8 is a graph showing the pH dependence of fluorescence intensity of PGA-AN5 and PGA-AN6 that are examples of the polymer in accordance with aspects of the invention in water.

FIG. 9 is a graph showing a fluorescence intensity ratio in a neutral-acidic region of PGA-AN that is an example of the polymer in accordance with aspects of the invention in water.

FIG. 10 is a graph showing a ratio of fluorescence intensities of various dyes in DMF and PBS.

### DESCRIPTION OF THE EMBODIMENTS

Embodiments of the invention will be explained below.

One aspect of the invention relates to a polymer having a fluorescent dye sensitive to a hydrophobic environment, wherein when a pH around the polymer decreases within a range of pH of equal to or greater than 5.5 and less than 7.4, a secondary structure changes from a random coil to a helix, and hydrophobicity around the fluorescent dye sensitive to the hydrophobic environment increases.

The "polymer" as referred to herein means a compound that has at least one of a synthetic polymer and a natural polymer, such as for example a polyamino acid or a sugar chain. The polymer in accordance with aspects of the invention may be determined as a compound with a weight-average molecular weight (Mw) of equal to or higher than 1000. The polymer is not particularly limited, provided that when a pH around the polymer decreases within a range of pH of equal to or greater than 5.5 and less than 7.4, a secondary structure changes from a random coil to a helix, and hydrophobicity around the fluorescent dye sensitive to the hydrophobic environment increases. The change of the secondary structure from a random coil to a helix will be hereinbelow referred to as a "helix transition".

Another aspect of the invention relates to a polymer having a fluorescent dye that is sensitive to a hydrophobic environment and a polyamino acid, wherein when pH around the polyamino acid decreases within a range of pH of equal to or greater than 5.5 and less than 7.4, a secondary structure changes from a random coil to a helix, and hydrophobicity around the fluorescent dye that is sensitive to the hydrophobic environment increases.

The phrase "pH ... decreases" as indicated above means that pH around the polymer in accordance with the invention changes from being more alkaline to being more acidic.

Without being limited to any particular theory herein, it is nonetheless believed that theoretical considerations based on the below-described mechanism in accordance with aspects of the invention demonstrate that the probability of the polymer in accordance with the invention taking a helix structure at pH 5.5 may be higher than the probability of taking a helix structure at pH 7.4. According to one aspect of the invention, the polymer may be selected such that the difference in the probability is significant (i.e., detectable when a group of compounds is observed). For example, where the probability of taking a helix structure at pH 5.5 is denoted by x% and the probability of taking a helix structure at pH 7.4 is denoted by y%, according to one aspect it can be said that a secondary structure changes from a random coil to a helix within a range of pH of equal to or greater than 5.5 and less than 7.4 if x is greater than y by 5 or more. In other words, at a pH of 7.4, a probability that the secondary structure of the polymer is a random coil structure is higher than a probability that the secondary structure of the polymer is a helix structure, and at a pH of 5.5, a probability that the secondary structure of the polymer is the helix structure is higher than a probability that the secondary structure of the polymer is the random coil structure.

Various methods that are well known in the related field can be used for confirming that the polymer in accordance with aspects of the invention has a helix structure or a random coil structure. Examples of such methods include circular dichroism (CD) measurements, infrared absorption spectrum measurements, fluorescence measurements, nuclear magnetic resonance (NMR) spectral analysis, neutron scattering, and mass spectroscopy. For example, the presence and content ratio of α-helix structure, β structure, and random coil structure can be estimated by measuring a CD spectrum in an ultraviolet region. More specifically, in a CD spectrum of a polyamino acid solution, an α-helix structure has negative maxima at 208 nm and 222 nm and a positive maximum at 192 nm, and a random coil structure has a negative maximum only in the vicinity of 200 nm. Therefore, a secondary structure can be analyzed from the CD spectrum.

As another method in an infrared absorption spectrum of a polyamino acid, a peptide bond absorption band is present in a frequency range of 1500 to 1700 cm⁻¹, and an oscillation band of amido I at 1600 to 1700 cm⁻¹ and amido II at 1500 to 1550 cm⁻¹ can be used for secondary structure estimation. In the α-helix structure, absorption bands are present at 1650 cm⁻¹ and 1546 cm⁻¹, and in the random coil structure, they are present at 1655 cm⁻¹ and 1535 cm⁻¹. The secondary structure can be analyzed by observing these absorption bands. CD spectrum measurements use a polyamino acid solution, but the infrared absorption spectrum measurements can be also performed with powder or film-like samples.

The expression "polymer having a fluorescent dye sensitive to a hydrophobic environment" as used herein means that some groups of the polymer are substituted with a fluorescent dye that is sensitive to a hydrophobic environment. The fluorescent dye sensitive to the hydrophobic environment in accordance with the aspects of the invention may be defined as a dye that increases in fluorescence intensity when the environment of the dye changes from a hydrophilic environment to a hydrophobic environment in a medium such as serum or water. Furthermore, in one embodiment, the fluorescent dye that is sensitive to the hydrophobic environment in accordance with aspects of the invention may increase the fluorescence intensity when the mobility of the dye itself changes from a high-mobility state to a low-mobility state.

The expression "polymer having a polyamino acid" as used herein means that the polymer includes one or more of a polyamino acid and a polyamino acid derivative. In accordance with aspects of the invention, a polyamino acid may be defined as a compound in which ten or more amino acids are linked by peptide bonds. A polyamino acid derivative means a polymer in which some polyamino acids or some of the groups of polyamino acids are substituted.

Examples of polyamino derivatives may include, but are not limited to, at least one of polyglutamic acid or polyasparagic acid that has a hydroxyalkylamino group, a sulfoalkylamino group, and a stearoyl group in a side chain, and polylysine in which a carboxyl is partially modified. A repeating unit of the main chain of the polyamino acid derivative may be a copolymer with a polymer comprising a monomer other than amino acid. The copolymer may be at least one of a block copolymer, a random copolymer, and a graft copolymer.

In a case where a large number of abnormal sites including tumors with low malignancy and inflammation sites are to be detected using the polymer in accordance with aspects of the present invention, the polymer may be selected such that the pH at which the secondary structure changes from a random coil to a helix may be equal to or greater than 5.7, such as equal to or greater than 6.0. In accordance with aspects of the present invention, from the standpoint of preventing the detection of a region with a somewhat decreased pH in a normal site as noise, it may be that the pH at which the secondary structure changes from a random coil to a helix is less than 6.9, such as less than 6.5.

### Mechanism of pH Variation Detection

The detection of pH variations by using the polymer in accordance with aspects of the invention is performed by using the decrease in pH around the polymer and increase in fluorescence intensity from the polymer observed when the polymer comes close to a detection object (for example, a low-pH site with a pH about 5.5) present in the test body. For example, the following mechanism may be used for detection.

In one embodiment, where the polymer in accordance with aspects of the invention comes close to the detection object (i.e., a detection object having a low pH), the pH around the polymer decreases. As a result of such decrease in pH, the secondary structure of the polymer changes (i.e., undergoes the helix transition). In addition, association and aggregation of the polymers can occur. As a result, a hydrophobic environment may be formed around at least some of the fluorescent dyes sensitive to hydrophobic environment, and the mobility of the dye itself decreases. Therefore, the intensity of fluorescence emitted from the dye can increase. In other words, the decrease in pH can be detected as the increase in fluorescence intensity. Furthermore, the apparent molecular weight of the polymer in accordance with aspects the invention may increase due to association or aggregation that accompanies the helix transition, and the diffusion rate may also decrease. As a result, the polymer may relatively easily remain in the low-pH region.

As an example, in a case where an acidic site of a tumor or an inflammation in a living body is to be detected, a polymer having a fluorescent dye sensitive to a hydrophobic environment and a polyglutamic acid can be advantageously used. Polyglutamic acid is soluble in water in a random coil state in a physiological pH region (pH: about 7.4), but in a low-pH region, a helix transition (i.e., variation in conformation) occurs and the secondary structure changes to a helix structure. Where pH further decreases, the acid becomes insoluble in water. Thus, in the physiological pH region, a fluorescent dye sensitive to hydrophobic environment is in a hydrophilic environment in which the dye is in contact with a relatively large number of water molecules. In addition, the dye itself has a relatively high mobility. As a result, the intensity of fluorescence from the dye is suppressed. By contrast, in a low-pH region, polyglutamic acid assumes a helix structure and the helixes form associations or aggregations. As a result, the fluorescent dye sensitive to the hydrophobic environment may be located in a hydrophobic environment formed inside the helix structure, or inside the association, and the mobility of the dye itself may be restricted. As a result, the fluorescence intensity increases, and the decrease in pH can be thus detected. In other words, in a tumor or inflammation site (pH 5 to 6), the fluorescence intensity increases over that in the normal site (pH: about 7.4). Therefore, the tumor or inflammation site can be detected due to the increase in the fluorescence intensity. In other words, according to one aspect of the invention, fluorescence intensity of the probe at a pH of 5.5 is higher than fluorescence intensity of the probe at a pH of 7.4 resulting from a change of the secondary structure of the polymer from a random coil structure to a helix structure.

The increase in hydrophobicity as used in the expression "the increase in hydrophobicity around the fluorescent dye sensitive to hydrophobic environment" can be confirmed by various methods known in the related art, for example, by fluorescence measurements and nuclear magnetic resonance spectral analysis. In the fluorescence measurements, the fluorescent dye sensitive to the hydrophobic environment that is the object may be dissolved in various polar solvents, for example, one or more of water, dimethylformamide (DMF), dimethylsulfoxide (DMSO), ethanol, methanol, chloroform, and hexane, and the dependence of fluorescence variation on the solvent can be observed. For example, in a case where the fluorescent dye sensitive to hydrophobic environment is anilinonaphthalene, the observations may show that in a hydrophobic solvent such as hexane, the fluorescence wavelength shifts to a short wavelength region and fluorescence intensity rises, whereas in a hydrophilic solvent such as water, the fluorescence wavelength shifts to a long wavelength region and fluorescence intensity decreases.

The fluorescence intensity of a polyamino acid to which the fluorescent dye sensitive to hydrophobic environment has been bonded may then be measured. Hydrophilicity and hydrophobicity around the fluorescent dye sensitive to the hydrophobic environment that has been bonded to the polyamino acid can be estimated by the obtained fluorescence intensity and the dependence of variation in the fluorescence intensity on the solvent that has been measured in advance. For example, an increase in fluorescence intensity of a polyamino acid having anilinonaphthalene bonded thereto indicates that hydrophobicity around anilinonaphthalene has risen, and a decrease in fluorescence intensity indicates that hydrophobicity around anilinonaphthalene has decreased. Furthermore, hydrophobicity around the dye can be also evaluated by observing the broadening of an NMR signal peak that follows the decrease in mobility of the dye in NMR spectral analysis method. Thus, where hydrophobicity around the dye increases, the dyes are aggregated together by hydrophobic interaction and mobility of the dye decreases. As a result, the peak of NMR signal from the dye becomes broader.

According to one embodiment, a polymer with a fluorescence intensity that is higher in an environment with a pH of about 5.5 in the test body, than in an environment with a pH of about 7.4, can be used as the polymer in accordance with aspects of the invention. Examples of the test body include blood serum and water.

With consideration for the above-described information, an example of an embodiment of the polymer in accordance with aspects of the invention will be described below.

### Polyamino Acid

Where the polymer in accordance with aspects of the invention has a polyamino acid, the polymer may be selected such that the secondary structure of the polyamino acid changes from a random coil to a helix within a range of pH of equal to or greater than 5.5 and less than 7.4, when the pH around the polymer decreases. Therefore, the polyamino acid may have a group that becomes a hydrophobic group when the pH around the polymer is greater than 5.5 and less than 7.4, and becomes a hydrophilic group when the pH is equal to or greater than 7.4. A carboxyl group is an example of such a group. Dissociative groups such as an amino group, a guanidine group, a phenyl group, an imidazole group, a phosphate group, a thiol group, and a sulfone group may be also included therein. The type and content ratio of these groups can be appropriately selected so that when the pH around the polymer decreases, the secondary structure of the polymer changes from a random coil to a helix, and hydrophobicity around the fluorescent dye that is sensitive to the hydrophobic environment rises, when the pH is within a range of equal to or greater than 5.5 and less than 7.4.

Examples of polyamino acids include, but are not limited to, at least one of homopolymers of glutamic acid, asparagic acid, histidine, arginine, tyrosine, and lysine, such as at least one of a glutamic acid homopolymer, such as polyglutamic acid, and an asparagic acid homopolymer. The polyamino acid may also contain a plurality of the aforementioned homopolymers. Furthermore, in one embodiment any polyamino acid may be used, provided that the secondary structure changes from a random coil to a helix within a range of pH of equal to or greater than 5.5 and less than 7.4, and hydrophobicity around the fluorescent dye that is sensitive to the hydrophobic environment rises when the pH around the polymer decreases. Therefore, one or more of random polymers, block copolymers, and graft polymers that include at least one of glutamic acid, lysine, asparagic acid, histidine, arginine, tyrosine and the like as constituent elements may also be used.

Furthermore, in one embodiment the polymer is selected such that the secondary structure of the polyamino acid changes when the pH changes, and from the standpoint of solubility in water and low viscosity, a number-average molecular weight of the polyamino acid may be from 20 to 1000. Where the number-average molecular weight is less than 20, secondary structure variations may hardly occur in the polyamino acid and, at the same time, a hydrophobic environment may be difficult to form around the dye.

Where the polymer in accordance with aspects of the invention and a fluorescent probe are used in a living body, they can be decomposed by enzymes present in the living body, and accordingly the polymer and fluorescent probe may be selected to have little or no toxicity or antibody activity with respect to a living body. From this standpoint, in one embodiment a polyamino acid that has good biocompatibility, demonstrates a helix transition within a range of pH of equal to or greater than 5.5 and less than 7.4, and has a degree of polymerization of 163 to 434 may be selected as the polyamino acid.

From the standpoint of biodegradability, in one embodiment L-modifications of the aforementioned amino acids may be selected, but D-modifications or mixtures of L-modifications and D-modifications may be also used. Non-natural amino acids may be also used. A non-natural amino acid or a D-modification amino acid residue can also be introduced in the polymer with the object of extending a half life of the polymer in blood.

Furthermore, an antibody, a fragmented antibody, or a receptor-bounded molecule may be bonded to the polymer in accordance with aspects of the invention. This may allow the target to be detected more specifically.

A large number of the above-described polyamino acids are commercially available, and a person skilled in the art can easily procure and use an appropriate polyamino acid. Furthermore, a polyamino acid can be easily synthesized by a successive peptide liquid-phase synthesis method or peptide solid-phase synthesis method, or an N-carboxylic acid anhydride (NCA) method.

### Fluorescent Dye Sensitive to Hydrophobic Environment

The fluorescent dye sensitive to hydrophobic environment used in accordance with the invention has already been defined hereinabove. In one embodiment, the fluorescent dye sensitive to hydrophobic environment that is used in accordance with aspects of the invention has little or no pH reactivity. In this embodiment, because the polyamino acid contained in the polymer in accordance with aspects of the invention has pH reactivity, where the fluorescent dye sensitive to the hydrophobic environment has pH reactivity, it can be difficult to perform adjustments such that the polymer in accordance with aspects of the invention demonstrates reactivity within a suitable pH range. Furthermore, in a case where the polymer in accordance with aspects of the invention is used as a fluorescence probe in a living body and fluorescence is observed from outside the living body, a fluorescent dye sensitive to the hydrophobic environment may be selected such that it has an absorption wavelength and an emission wavelength in a near-IR region. On the other hand, in a case where the polymer in accordance with aspects of the invention is used as a fluorescence probe inside a living body and the observations are performed inside the living body or the observation object is removed for observations to the outside of the living body, the fluorescent dye sensitive to the hydrophobic environment may be selected to have the absorption and emission wavelengths in the UV and visible range.

### Solvatofluorochromic dyes

Among the fluorescent dyes sensitive to hydrophobic environment, solvatofluorochromic dyes are known as dyes with a significant increase in fluorescence intensity. As will be described below in the examples, in accordance with aspects of the invention, the solvatofluorochromic dye is defined as a dye in which the fluorescence intensity in DMF (N, N-dimethylformamide) is higher by a factor of 20 or more than the fluorescence intensity in PBS (phosphate buffered saline).

Examples of solvatofluorochromic dyes include, but are not limited to, at least one of anilinonaphthalene, Nile Red, dansyl (dimethylaminonapthalenesulfonic acid), and nitrobenzoxadiazole (NBD), pyrene, and derivatives thereof. In one embodiment, anilinonaphthalene derivatives represented by Formula (1) below, and Nile Red derivatives represented by Formula (2) below, may be selected. Among them, anilinonaphthylmaleimide and hydroxyl group-modified Nile Red (DEAHB: 9-diethylamino-2-hydroxy-5H-benz[a]phenoxazin-5-one), for example, may be selected. R in Formula (1) and Formula (2) stands for a bonded active group such as a maleimide group and a hydroxyl group.

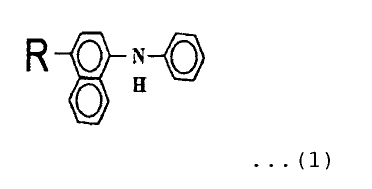

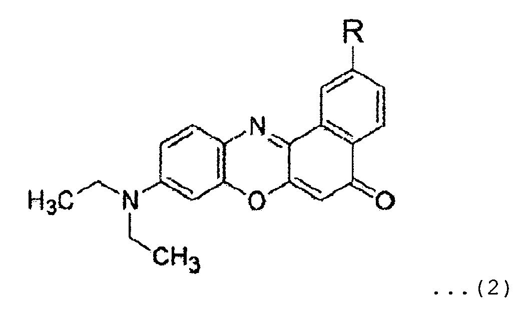

### Bonding of Fluorescent Dye Sensitive to the Hydrophobic Environment

The fluorescent dye sensitive to the hydrophobic environment can be bonded by a conventional well-known coupling reaction to some of the groups of the polymer to which the fluorescent dye sensitive to the hydrophobic environment is not yet bonded. For example, in a case where the group is a carboxyl group, a dye having at least one of an amino group, a thiol group, and a hydroxyl group that shows reactivity with respect to the carboxyl group can be coupled. In a case where the group is an amino group, a dye having at least one of a carboxyl group, a sulfonate group, a hydroxysuccinimide group, an aldehyde group, a thiol group, an isothiocyanate group, and a glycidyl group that shows reactivity with respect to the amino group can be coupled. In a case where the group is a hydroxyl group or a thiol group, a dye having at least one of a carboxyl group, a sulfonate group, a halogen, a disulfide, and a maleimide group that shows reactivity with respect to these groups can be coupled.

Furthermore, bonding of the polymer and the fluorescent dye sensitive to hydrophobic environment is not limited to direct bonding, and can also be performed, for example, via an appropriate spacer molecule. In one embodiment, a bifunctional short-chain alkane such as an alkanedithiol and alkanediamine, a bifunctional oligoethylene oxide chain, or an acid anhydride can be used as the spacer molecule. One end group of the spacer molecule typically may be bonded to a group of the polymer and the other end group of the spacer molecule may be bonded to a group of the dye. It goes without saying that bonding between the polymer and the fluorescent dye sensitive to hydrophobic environment and the type of spacer molecule used are not limited to the above-described ones, and can be appropriately selected by a person skilled in the art from a variety of bonding methods and spacers that can be used. Where the polymer in accordance with aspects of the invention is used inside a living body, the polymer may be selected to be soluble in water. Therefore, in one embodiment, the fluorescent dye sensitive to hydrophobic environment represented by Formulas (1) and (2) may be introduced at a ratio of 0.0005 to 0.03 with respect to the degree of polymerization of the entire polymer.

### Terminal Residue

Any residue may be bonded to the end group of the polymer, provided that the residue does not inhibit the ability to detect pH variation. For example, a group determined by a polymerization initiator, or a capping agent that is used when the polymer is synthesized, may be bonded. In a case where the polymer has a polyamino acid, for example, one or more of -H, -OCH₃, and -COCH₃ can be considered for the N terminal of the polyamino acid. For example, one or more of -NH₂, -OH, -NH(CH₂)_{z}CH₃ (here Z is an integer of 2 to 5) can be considered for the C terminal.

### Examples of Polymers

Examples of embodiments of the polymer in accordance with aspects of the invention include the polymers represented by the following Formulas (3) and (4). These polymers may be obtained by introducing anilinonaphthylimide and hydroxyl group-modified Nile Red in polyglutamic acid. The number-average molecular weight (m + n) is within a range of 50 to 1000, and a dye modification ratio n/(m + n) is within a range of 0.0005 to 0.03.

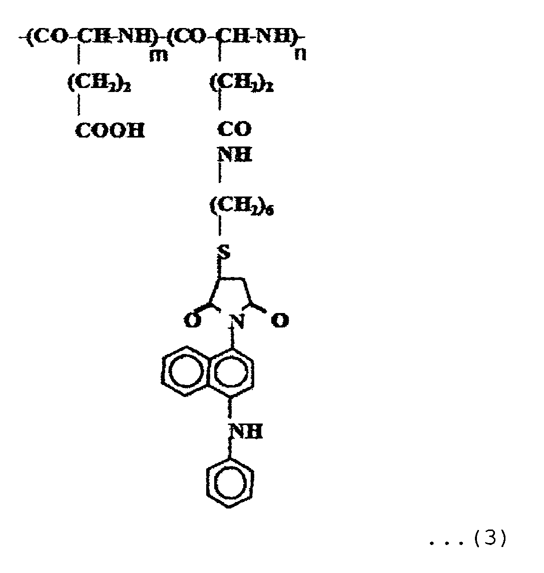

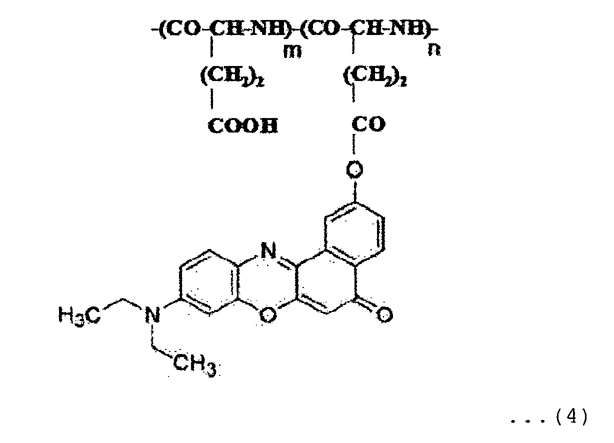

### Hydrogelling

The polymer in accordance with aspects of the invention may be hydrogelled by intermolecular crosslinking. For example, hydrogelling can be performed by bonding groups of the polymer, other than the fluorescent dye sensitive to hydrophobic environment, by using a multifunctional crosslinking agent. In one embodiment, this bonding can be performed in the same manner as the bonding of the polymer and the fluorescent dye that is sensitive to the hydrophobic environment. A diamine compound, a diol compound, and a diglycidyl ether can be used as the crosslinking agent. The polymer in accordance with the invention may also be molded into nanosize hydrogel particles by a well-known method that will be described hereinbelow in examples. For example, it is known that nanosize hydrogel particles can be obtained by a molding method using an inverted micelle as a nanosize reaction field. The crosslinking reaction and type of crosslinking agent are not limited to those described hereinabove, and a person skilled in the art can also appropriately select them from a variety of suitable crosslinking reactions and crosslinking agents. A nanogel-size particle of the polymer in accordance with the invention may find application as a pH-reactive fluorescence probe that can inhibit contact of the dye with impurities such as proteins, and can demonstrate high tumor retention ability due to the EPR effect.

### Applications of Polymer According to aspects of the Invention

In one embodiment, an application of the polymer in accordance with aspects of the invention is a fluorescence probe that can detect disease-related abnormal pH variations. For example, the polymer may be used as a fluorescence probe that can be fluorescence sensitive to pH decrease in locations (pH: about 5 to about 6) of inflammations or tumors. Another application is as a fluorescence probe that can be fluorescence sensitive to pH decrease in a tumor location (pH: about 5.5) with a high degree of malignancy. However, the application objects of the probe in accordance with aspects of the invention are not limited to these diseases.

In one embodiment, the polymer in accordance with aspects of the invention may also be suitable as a fluorescence probe for understanding intracellular transport and protein decomposition mechanism. For example, the polymer can be used as a fluorescence probe that can detect an acidic environment in an intracellular endosome and trace endocytosis. Such a probe may find applications in the field of, for example, cytobiology.

### EXAMPLES

An exemplary compound using polyglutamic acid as a polymer in accordance with aspects of the invention, and a preparation method therefor will be described in the following examples. A fluorescence probe can be relatively easily prepared by appropriately modifying or changing the starting materials, reagents, and reaction conditions with reference to these methods. It should be understood that methods for preparing the polymer in accordance with aspects of the invention are not limited to those described in examples below.

### EXAMPLE 1

### Synthesis of Polymer

### Example 1-1

### Succinimidization of poly-L-glutamic acid

A total of 1 g of a sodium salt of poly-L-glutamic acid (PGA; Mw 41,000, Peptide Research Institute) was dissolved in 100 mL of distilled water, then stirring was conducted, while dropwise adding hydrochloric acid, and desalinated PGA was precipitated. The obtained desalinated PGA was thoroughly washed with acetone, washed with ether, and then vacuum dried. The washed and dried PGA (150 mg) was then dissolved in 1 mL of anhydrous DMSO, N-hydroxysuccinimide (NHS; 13.5 mg) and 1-ethyl-3-dimethylaminopropylcarbodiimide hydrochloride (EDC; 22.2 mg) were added, and then shaking was conducted overnight at room temperature. A 10% succinimidization was conducted with respect to the entire COOH in the charging ratio. After the reaction, reprecipitation was performed using anhydrous acetone in order to remove the unreacted EDC and 1-ethyl-3-(3-dimethylaminopropyl) urea, which is a reaction after-product of NHS and EDC. Then, centrifugal separation was conducted and washing was further performed twice by decantation. Washing with hexane, ether substitution, and vacuum drying were then conducted to obtain succinimidized PGA.

### Example 1-2

### Modification of Succinimidized PGA with Anilinonaphthylimide

The succinimidized PGA (150 mg) prepared according to the above-described Example 1-1 was dissolved in anhydrous DMSO (5 mL). Then, N-(1-anilinonaphthyl-4-)-maleimido (anilinonaphthylimide: ANM) (21 mg) and 6-amino-1-hexanethiol (11 mg) were mixed, the DMSO solution, 1.5 mL, that was stirred overnight was added, and stirring was conducted overnight at room temperature in a dark room. The reaction solution was reprecipitated using acetone was then washed twice by decantation. Washing with hexane, ether substitution, and vacuum drying were then conducted to obtain 136 mg of anilinonaphthylmaleimide-modified PGA (PGA-AN1) (yield 90%). The modification ratio (n/n + m) of the AN group with respect to the entire COOH of the PGA was confirmed to be 0.006 by measuring the absorbance (350 nm) of and anilinonaphthyl (AN) group of the obtained PGA-AN1. The structural formula of the obtained PGA-AN1 is shown in Formula 3 above. In this formula m was 316 and n was 1.9.

Modification with anilinonaphthylmaleimide was also conducted in the same manner with respect to succinimidized PGA with different molecular weights that were prepared according to Example 1-1 (PGA-AN2 and PGA-AN3). A system having anilinonaphthylmaleimide introduced therein in an about fivefold amount was also prepared (PGA-AN4). Systems using 11-amino-1-undecanethiol instead of 6-amino-1-hexanethiol were also prepared (PGA-AN5 and PGA-AN6). The molecular weight, degree of polymerization, AN modification ratio, and linker alkyl chain length of the synthesized PGA-AN1-6 are shown in Table 1.

**Table 1. Synthesized polymers and AN modification ratios**

| Polymer | Molecular weight | Degree of polymerization (n + m) | AN modification ratio (n/n + m) | Linker alkali chain length |
|---|---|---|---|---|
| PGA-AN1 | 41,000 | 318 | 0.006 | 6 |
| PGA-AN2 | 21,100 | 163 | 0.001 | 6 |
| PGA-AN3 | 56,000 | 434 | 0.0007 | 6 |
| PGA-AN4 | 41,000 | 318 | 0.027 | 6 |
| PGA-AN5 | 21,100 | 163 | 0.002 | 11 |
| PGA-AN6 | 56,000 | 434 | 0.006 | 11 |

### Example 1-3

### Modification of Succinimidized PGA with 9-Diethylamino-2-hydroxy-5H-benz[a]phenoxazin-5-one

The succinimidized PGA (150 mg) prepared according to Example 1-1 was dissolved in anhydrous DMSO (5 mL), 9-Diethylamino-2-hydroxy-5H-benz[a]phenoxazin-5-one (DEAHB), 20 mg, which is a Nile Red derivative, was mixed with the solution, and stirring was conducted overnight at room temperature in a dark room. The reaction liquid was reprecipitated with acetone and washed twice by decantation. Washing with hexane, ether substitution, and vacuum drying were then conducted to obtain 116 mg of DEAHB-modified PGA (PGA-NR) (yield 76%). The modification ratio of the DEAHB group with respect to the entire COOH of the PGA was confirmed to be 0.002 by measuring the absorbance (600 nm) of and DEAHB group of the obtained PGA-NR. The structural formula of the obtained PGA-NR is shown in Formula 4 above. In this formula m was 317 and n was 0.6.

### Example 1-4

### Preparation of Hydrogel Particles

A total of 10 mg of PGA-AN1 was dissolved in 1 mL of distilled water, and 0.1 mL of ethylene glycol glycidyl ether (EGDGE) was added as a crosslinking agent to the solution. A total of 200 mL of 0.05 M n-hexane solution of sodium bis(2-ethylhexyl)sulfosuccinate (AOT) was then immediately added, ultrasound irradiation was conducted, and then stirring was performed for 5 days at room temperature. The solvent was then removed with an evaporator and vacuum drying was then conducted overnight. Washing was conducted three times with acetone/methanol (9/1) and ether substitution as performed, followed by vacuum drying. As a result, hydrogen particles PGA-AN-Gel of PGA-AN1 were obtained. The obtained PGA-AN-Gel was dispersed in water and filtered through a 0.45-micron filter. The formation of particles with a diameter of about 100 nm was confirmed by a dynamic light scattering (DLS) method.

### EXAMPLE 2

### Evaluation of Polymer

### Example 2-1

### Measurement of pH Dependence of Fluorescence Intensity of PGA-AN in Water

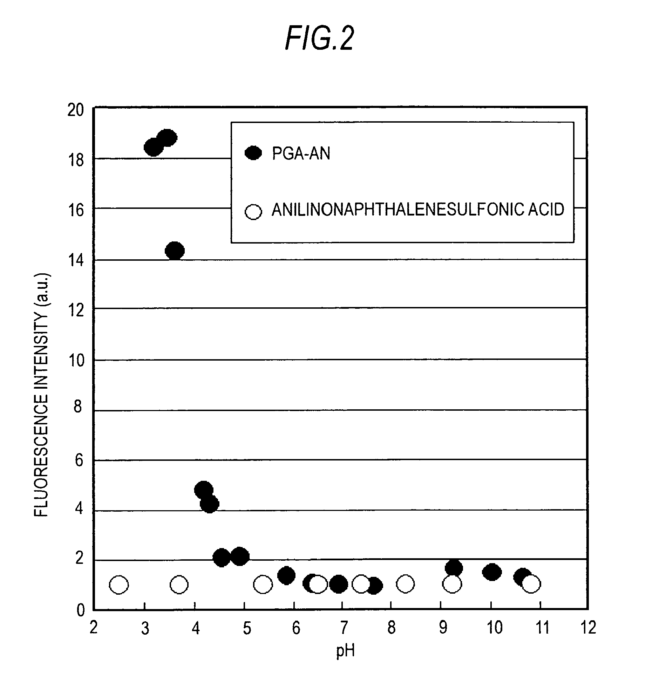

A total of 1 mL of aqueous solution of PGA-AN1 (1 mg/mL) was introduced in a quartz cell and fluorescence measurements were conducted at room temperature. Then, 6N HCl and 6N NaOH were used to adjust pH. The excitation wavelength was 350 nm, and a value of the maximum fluorescence wavelength at which the fluorescence intensity appeared at 440 to 470 nm was measured. The variations in fluorescence intensity occurring when the pH of the aqueous solution of PGA-AN1 is changed are shown by black circles in FIG. 2. The fluorescence intensity was observed to increase with the decrease in pH. By contrast, at a pH equal to or greater than 7, the fluorescence intensity did not change. At pH equal to or less than 3, the probe was observed to precipitate. The fluorescence intensity at pH 4 was increased by a factor of about 10 with respect to that at pH 7.4. The results obtained in measuring the pH dependence of fluorescence intensity of independent dye, that is, anilinonaphthalenesulfonic acid, in water are plotted as a comparative example in FIG. 2. As follows from FIG. 2, no pH dependence of fluorescence intensity was observed. The above-described results demonstrate that the fluorescence probe PGA-AN1 in which an anilinonaphthyl group, which is a fluorescent dye sensitive to a hydrophobic environment, is introduced in a group of polyglutamic acid demonstrates the increase in fluorescence intensity at pH less than 7.4. Therefore, this probe can be used for determining the decrease in pH from the physiological pH 7.4.

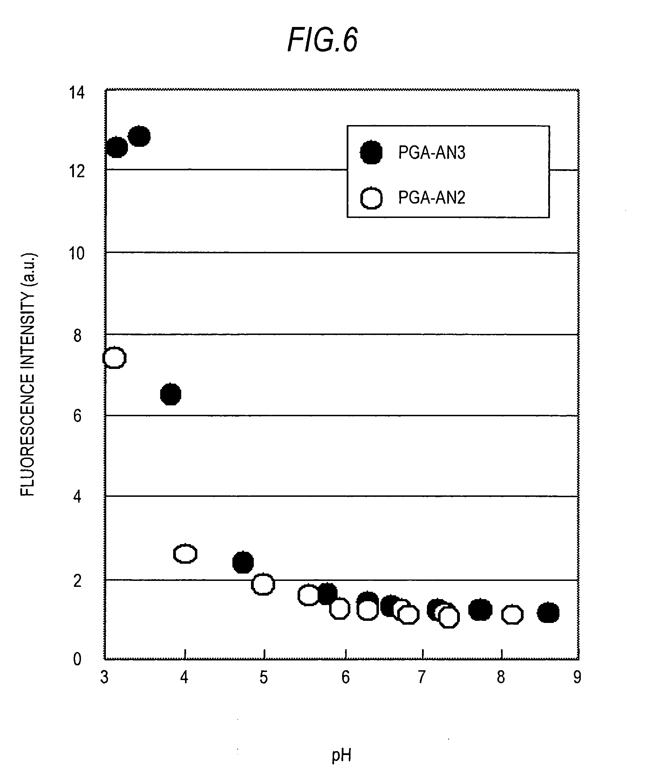

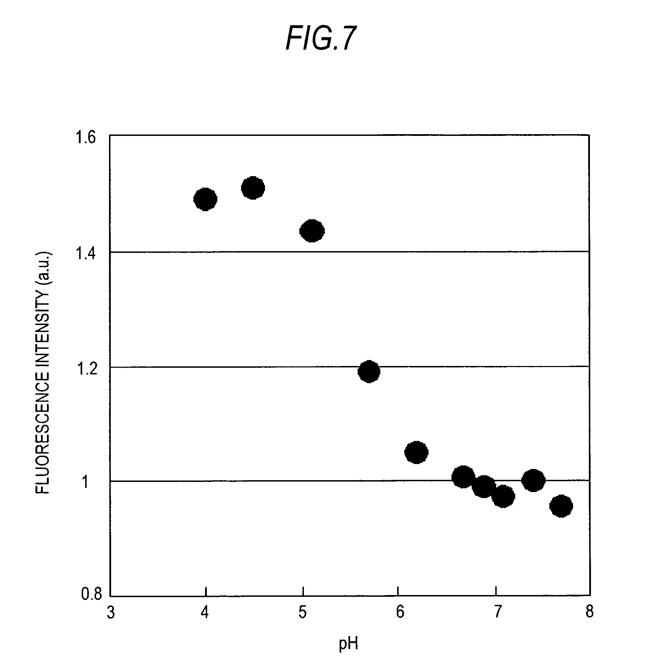

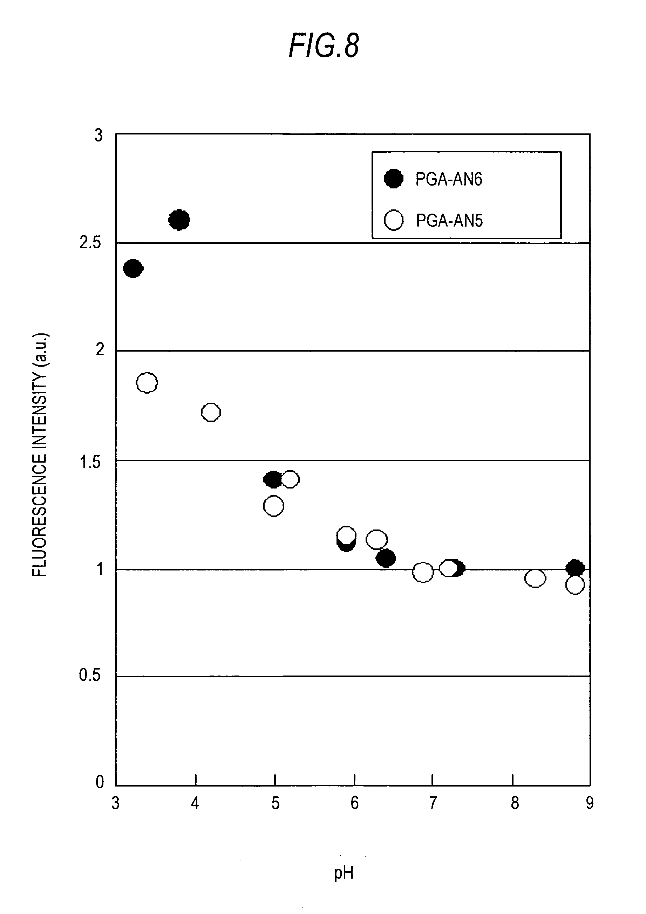

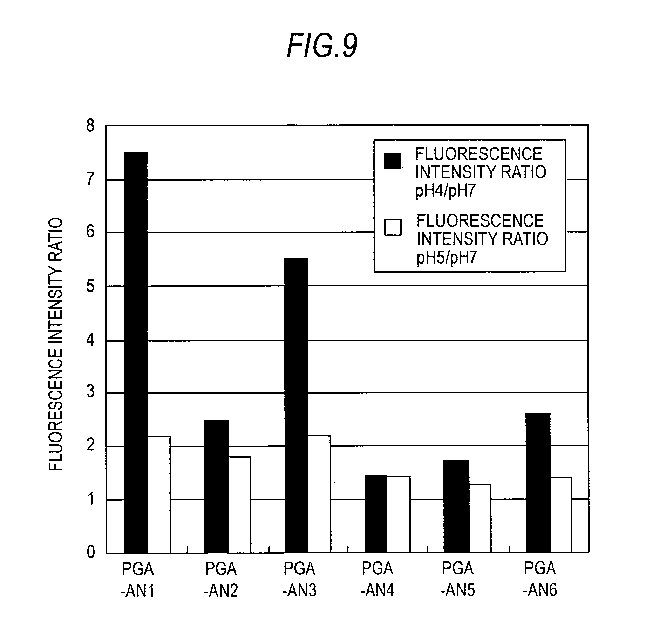

Likewise, the pH dependence of fluorescence intensity was also measured for the fluorescence probes PGA-AN2 to 6. The results relating to PGA-AN2 and 3 are shown in FIG. 6, the results relating to PGA-AN4 are shown in FIG. 7, and the results relating to PGA-AN5, 6 are shown in FIG. 8. In all the cases, the increase in the fluorescence intensity was observed to decrease with the decrease in pH. In FIG. 9, the fluorescence intensity ratio at pH 7 and pH 5 and the fluorescence intensity ratio at pH 7 and pH 4 are shown for aqueous solutions of PGA-AN1 to 6. FIG. 9 shows that PGA-AN1, 2 and 3 demonstrate the fluorescence intensity ratios higher than those demonstrated by PGA-AN4, 5, and 6. The molecular weight, AN modification ratio, and length of alkyl linker between the PGA backbone and the AN group can be considered to affect the variations in secondary structure in the acidic region.

### Example 2-2

### Measurement of pH Dependence of Fluorescence Intensity of PGA-NR in Water

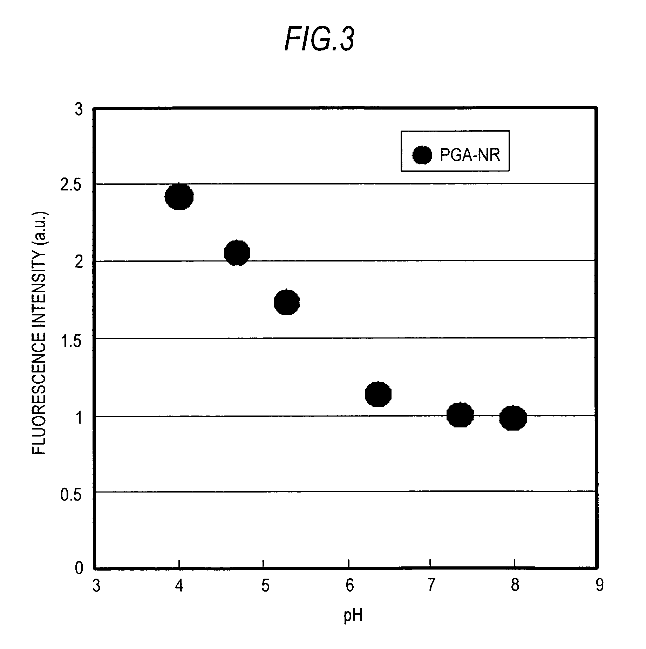

Fluorescence measurements of an aqueous solution of PGA-NR (1 mg/mL) were conducted in the same manner as in Example 2-1. The excitation wavelength was 550 nm, and a value of the maximum fluorescence wavelength at which the fluorescence intensity appeared at 600 to 660 nm was measured. The variations in fluorescence intensity occurring when the pH of the aqueous solution of PGA-NR is changed are shown in FIG. 3. The fluorescence intensity was observed to increase with the decrease in pH. By contrast, at pH equal to or greater than 7, the fluorescence intensity did not change. The fluorescence intensity at pH 4 was increased by a factor of about 2.4 with respect to that at pH 7.4. Because DEAHB is not soluble in water, fluorescence measurements of the independent dye were not performed. The above-described results demonstrate that the polymer PGA-NR in which the Nile Red derivative, which is a solvatofluorochromic dye, is introduced in a group of polyglutamic acid demonstrates the increase in fluorescence intensity at pH less than 7.4. Therefore, this probe can be used for determining the decrease in pH from the physiological pH 7.4. Furthermore, the excitation wavelength and fluorescence wavelength were at a longer wavelength side (equal to or greater than 550 nm) than in PGA-AN, and the fluorescence probe was found to have high utility in applications to cells and living body tissues.

### Example 2-3

### Measurement of pH Dependence of Fluorescence Intensity of PGA-AN-Gel in Water

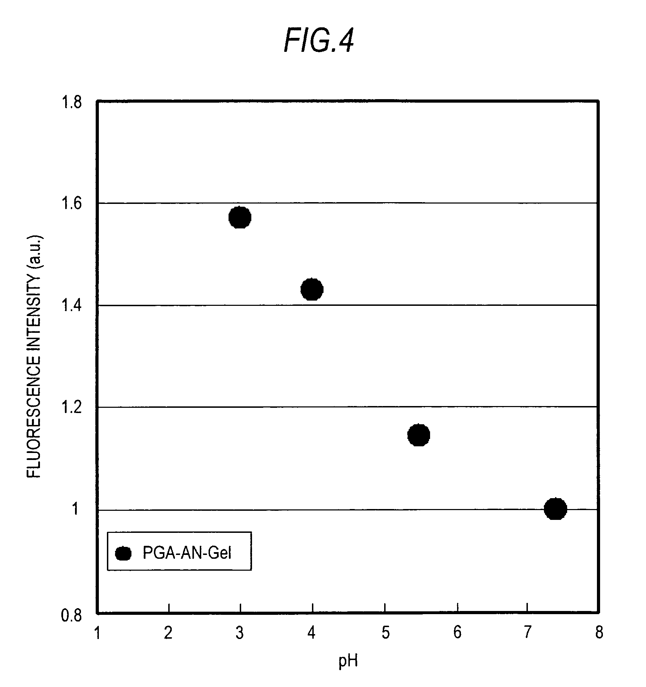

Fluorescence measurements of an aqueous solution of PGA-AN-Gel (1 mg/mL) were conducted in the same manner as in Example 2-1. The excitation wavelength was 350 nm, and a value of the maximum fluorescence wavelength at which the fluorescence intensity appeared at 440 to 470 nm was measured. The variations in fluorescence intensity occurring when the pH of the aqueous solution of PGA-AN-Gel is changed are shown in FIG. 4. The fluorescence intensity was observed to increase with the decrease in pH. The fluorescence intensity at pH 3 was increased by a factor of about 1.6 with respect to that at pH 7.4.

The above-described results demonstrate that the fluorescence probe obtained by nanosize hydrogelling the PGA-AN can be used, similarly to the fluorescence probe that has not be hydrogelled, as a fluorescence probe capable of determining the decrease in pH from the physiological pH 7.4.

### Example 2-4

### Measurement of pH Dependence of Fluorescence Intensity of PGA-AN1 in 10% Blood Serum

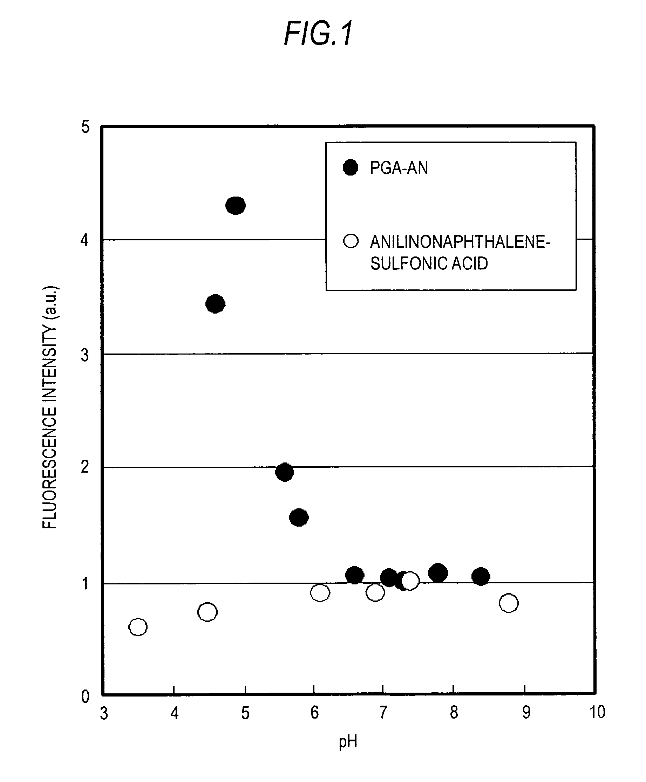

Fluorescence measurements were conducted in the same manner as in Example 2-1 in a phosphate buffered saline (PBS) (probe concentration 1 mg/mL) of PGA-AN1, the solution containing 10% fetal calf serum (FCS). The excitation wavelength was 350 nm, and a value of the maximum fluorescence wavelength at which the fluorescence intensity appeared at 440 to 470 nm was measured. The variations in fluorescence intensity occurring when the pH of the aqueous solution of PGA-AN1 is changed are shown by black circles in FIG. 1. The fluorescence intensity was observed to increase with the decrease in pH. By contrast, at pH equal to or greater than 7, the fluorescence intensity did not change. At pH equal to or less than 4.6, the probe was observed to precipitate. The fluorescence intensity at pH 5 was increased by a factor of about 4 with respect to that at pH 7. The pH dependence of fluorescence intensity of the independent dye, that is, anilinonaphthalenesulfonic acid, is plotted by white circles as a comparative example in FIG. 1. As follows from FIG. 1, no pH dependence of fluorescence intensity was observed. The above-described results demonstrate that the polymer PGA-AN1 in which an anilinonaphthyl group, which is a solvatofluorochromic dye, is introduced in a side chain of polyglutamic acid can be used as a fluorescence probe that maintains pH reaction function even in 10% blood serum.

### Example 2-5

### Evaluation of Dependence of Fluorescence Intensity of PGA-AN1 on Blood Serum Concentration

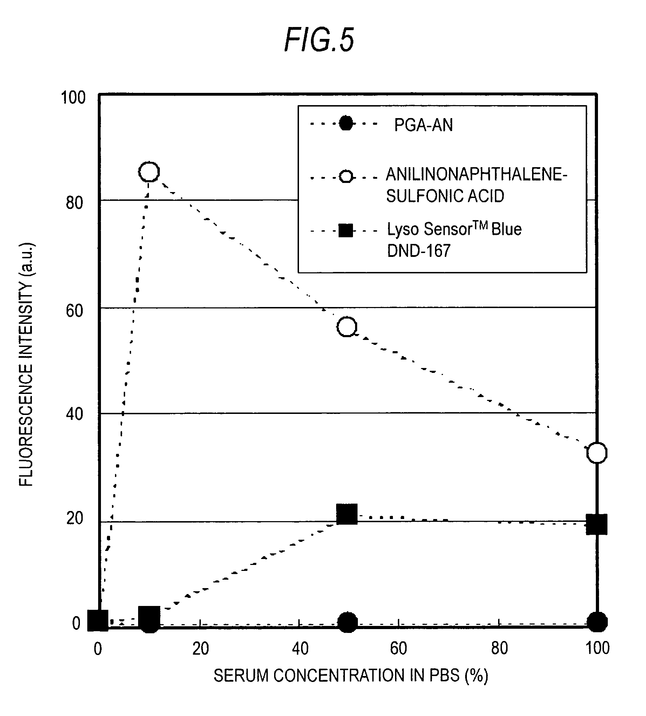

The interaction of PGA-AN1 and serum protein was evaluated by measuring the dependence of fluorescence intensity of PGA-AN1 on blood serum concentration. An independent dye, that is, anilinonaphthalenesulfonic acid, was used as a low-molecular probe for comparison. Furthermore, LysoSensor™ Blue DND-167 (manufactured by Invitrogen; excitation wavelength 373 nm, fluorescence wavelength 425 nm) in which the fluorescence intensity increases in relation to pH when the environment becomes acidic was used as a low-molecular pH-reactive probe for comparison. A solution of each probe was added to PBS solutions of 0, 10, and 100% FCS and then sufficiently mixed to measure the fluorescence intensity. The plot in FIG. 5 represents the relationship between the FCS concentration and fluorescence intensity normalized by the fluorescence intensity in serum-free PBS solution. In the low-molecular probe, the fluorescence intensity increased with the increase in serum concentration. This increase in fluorescence intensity is apparently due to bonding of the probe induced by hydrophobic interaction with serum protein. By contrast, no significant change in fluorescence intensity was observed in PGA-AN1 even in 100% FCS, which indicated that backbone PGA inhibits interaction of the dye and serum protein. The above-described results indicate that in the fluorescence probe in accordance with the present invention, the backbone polypeptide plays two roles: providing pH reactivity and inhibiting dye - protein interaction.

### Example 3-1

### Definition of Solvatofluorochromic dye in Accordance with Aspects of the Invention

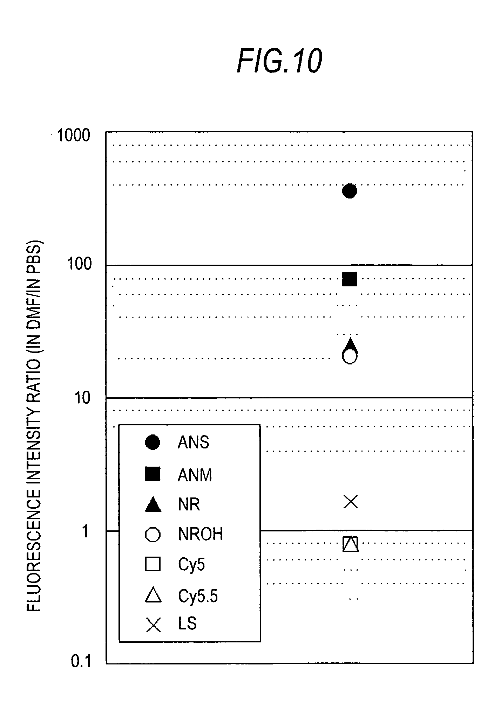

A total of seven fluorescence dyes: magnesium anilinonaphthalene sulfonate (ANS) and anilinonaphthylimide (ANM), which are anilinonaphthalene derivatives, Nile Red (NR) and 9-diethylamino-2-hydroxy-5H-benz[a]phenoxazin-5-one (NROH), which are Nile Red derivatives, and Cy5, Cy5.5, and LysoSensor^{™} Blue DND-167 (LS) were dissolved in DMF and PBS and fluorescence intensity thereof was measured. For all the dyes, 0.3 to 1 mM DMSO solutions were used as stock solutions and measurement samples were obtained by diluting them with PBS or DMF by a factor of 1000. The sample concentration was 3 µM for ANS, 1 µM for ANM, NR, NROH, Cy5, and LS, and 0.6 µM for Cy5.5. The excitation wavelength ex / fluorescence wavelengths emDMF,emPBS, all indicated in nm, were as follows: ANS (ex350 / em468,em520), ANM (ex350 / em456,em520), NR (ex550 / em622,em660), NROH (ex550 / em616,em659), Cy5 (ex650 / em677,em665), Cy5.5 (ex678 / em710,em690), and LS (ex373 / em439,em429). The results are shown in FIG. 10. As shown in the figure, the solvatofluorochromic dye in accordance with aspects of the invention has a fluorescence intensity in DMF that is 20 or more times that in PBS.

The examples thus demonstrate that a novel polymer probe can be provided that demonstrates a higher fluorescence intensity when placed in an environment with pH of about 5.5 than when placed in an environment with pH about 7.4, and that generates a relatively high fluorescence intensity.

The examples also demonstrate that a polymer and fluorescence probe in accordance with aspects of the invention can be used for relatively accurate detection of highly malignant tumor sites (i.e., those with a pH of about 5.5).

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A polymer having a fluorescent dye sensitive to a hydrophobic environment, wherein when a pH around the polymer decreases within a range of pH of equal to or greater than 5.5 and less than 7.4, a secondary structure of the polymer changes from a random coil to a helix, and hydrophobicity around the fluorescent dye sensitive to the hydrophobic environment increases.

2. A polymer having a fluorescent dye sensitive to a hydrophobic environment and a polyamino acid, wherein when a pH around the polyamino acid decreases within a range of pH of equal to or greater than 5.5 and less than 7.4, a secondary structure of the polymer changes from a random coil to a helix, and hydrophobicity around the fluorescent dye sensitive to the hydrophobic environment increases.

3. The polymer according to claim 2, wherein the polyamino acid is polyglutamic acid.

4. The polymer according to claim 1, wherein the fluorescent dye sensitive to the hydrophobic environment is a solvatofluorochromic dye.

5. The polymer according to claim 4, wherein the solvatofluorochromic dye is at least one of anilinonaphthalene, Nile Red, dansyl, nitrobenzoxadiazole, pyrene, and derivatives thereof.

6. A fluorescence probe for detecting a lesion site of a living body, comprising the polymer according to claim 1.

7. The polymer according to claim 1, wherein when a pH around the polymer decreases within a range of pH of equal to or greater than 5.5 and less than 6.9, the secondary structure of the polymer changes from a random coil to a helix.

8. The polymer according to claim 2, wherein when a pH around the polymer decreases within a range of pH of equal to or greater than 5.5 and less than 6.9, the secondary structure of the polymer changes from a random coil to a helix.

9. A polymer comprising a fluorescent dye sensitive to a hydrophobic environment, wherein at a pH of 7.4, a probability that a secondary structure of the polymer is a random coil structure is higher than a probability that the secondary structure of the polymer is a helix structure, and at a pH of 5.5, a probability that the secondary structure of the polymer is the helix structure is higher than a probability that the secondary structure of the polymer is the random coil structure.

10. A fluorescence probe comprising a polymer and a fluorescent dye sensitive to a hydrophobic environment, wherein fluorescence intensity of the probe at a pH of 5.5 is higher than fluorescence intensity of the probe at a pH of 7.4 resulting from a change of a secondary structure of the polymer from a random coil structure to a helix structure.

11. The fluorescence probe according to claim 10, wherein the polymer comprises at least one of a sugar chain, a polyamino acid, and derivatives thereof.

12. The fluorescence probe according to claim 10, wherein hydrophobicity around the fluorescent dye sensitive to the hydrophobic environment increases with the change of the secondary structure of the polymer from the random coil structure to the helix structure.
